# EUROPEAN PATENT SPECIFICATION

(11) **EP 2 460 559 B1**
(45) Date of publication and mention of the grant of the patent: **08.07.2015**
(21) Application number: 11009608.8
(22) Date of filing: 06.12.2011
(51) Int. Cl.: A61N 1/36, A61N 1/378

(54) **Living tissue stimulation apparatus**
Vorrichtung zur Stimulation von Lebendgewebe
Appareil de stimulation de tissus vivants

(30) Priority: 06.12.2010 JP 2010271889; 06.12.2010 JP 2010271890
(43) Date of publication of application: 06.06.2012
(73) Proprietor: NIDEK CO., LTD, Gamagori-shi Aichi (JP)
(72) Inventor: Shodo, Kenzo, Gamagori Aichi (JP)
(74) Representative: Hoefer & Partner Patentanwälte mbB

(56) References cited:
- WO-A1-03/077191
- JP-A- 2010 187 747
- US-A- 4 612 934
- US-A1- 2002 161 417
- US-B1- 6 458 157
- US-B1- 7 831 309

## Description

### BACKGROUND

The present invention relates to a living tissue stimulation apparatus which electrically stimulates a portion of living tissue.

A living tissue stimulation apparatus which implants a stimulating electrode for living tissue (hereinafter, denoted as an "electrode") in the body and electrically stimulates a portion of the living tissue has been studied. For example, living tissue stimulation apparatuses such as an artificial middle ear for transmitting sound vibrations to an auditory ossicles of a patient, a cardiac pacemaker which is implanted in the chest of a patient and suppresses occurrence of an irregular pulse by applying electrical stimulation to the heart, and a visual regeneration assisting apparatus which electrically stimulates cells constituting a retina by an electric stimulation pulse signal (electric charge) output from electrodes and attempts visual regeneration (for example, refer to JP2010187747 and WO03/077191) are known.

The visual regeneration assisting apparatus of JP2010187747 is configured by combining an external device which images a picture of the outside world and supplies power to the entire apparatus and an internal device which applies electric stimulation to the retina based on a signal (power) from the external device, the external device is mounted outside the body of a patient, and the internal device is implanted inside the body of the patient. In addition, the internal device is configured of a receiving unit which receives power (information) from the external device and a stimulation unit in which a plurality of electrodes are formed, and the receiving unit and the stimulation unit are electrically connected to each other by cables.

Incidentally, it is preferable that the internal device be as small as possible in order to decrease burden on a patient, and the diameter of the cable which transmits power or various signals is also required to be small. However, since the cable in the related art requires a conducting wire for supplying alternating-current power, a conducting wire for transmitting a control signal, and a conducting wire for transmitting a clock signal, or the like, respectively, there is a limit on decreasing the diameter of the cable.

Moreover, in this kind of device, a circuit configuration generating the clock signal which becomes a time reference for correctly operating the stimulation unit is separately required. For example, in the device of WO03/077191, the clock signal is output by using a transmitter. On the other hand, in order to stably operate the living tissue stimulation apparatus implanted inside of the body for a long time, it is necessary to suppress unnecessary power consumption as much as possible and use the power supplied from a power supply efficiently, and it is preferable that the power consumption of the configuration for operating the circuits such as the transmitter be suppressed.

Document US4612934 discloses an electronic tisue stimulator device. Using an internal voltage source, programming data are transmitted as a modulated signal on a carrier wave.

### SUMMARY

According to the present invention, a living tissue stimulation apparatus in which power is efficiently used by a simple configuration can be provided.

In order to solve the above described problems, the present invention is characterized in that it includes the following configurations.
(1) A living tissue stimulation apparatus as defined by claim 1.
(2) The living tissue stimulation apparatus according to (1) further comprising a cable configured to electrically connect the periodic modulation circuit to the clock signal generation portion,
   wherein the cable includes a first conducting wire pair configured to transmit the periodic-modulated wave output from the periodic modulation circuit, and a second conducting wire configured to transmit the electric stimulation pulse signal, output from the electrodes, for electrically stimulating the living body.
(3) The living tissue stimulation apparatus according to (2), wherein
   the clock signal generation portion includes:
   a comparator configured to detect the waveform period for each period of the periodic-modulated wave generated by the periodic modulation circuit and generate a clock signal based on the waveform period;
   a plurality of period-voltage conversion circuits configured to convert the waveform period for each period of the periodic-modulated wave detected by the comparator into a voltage;
   a plurality of window comparators configured to compare the voltages which are continuously output from the two period-voltage conversion circuits and output a signal; and
   a signal generation portion configured to extract the clock signal based on the output signal from the window comparators.
(4) The living tissue stimulation apparatus according to (3), wherein at least three period-voltage conversion circuits are provided and at least three window comparators are provided.
(5) The living tissue stimulation apparatus according to (4), wherein the periodic-modulated wave output from the periodic modulation circuit is intermittently supplied to the stimulation unit via the first conducting wire pair when the state change of the stimulation unit is performed.
(6) The living tissue stimulation apparatus according to (5), further comprising a power transmission unit configured to electrically connect the power supply unit to the stimulation unit,
   wherein the stimulation unit is driven by the alternating-current power intermittently supplied from the power supply unit via the power transmission unit.
(7) The living tissue stimulation apparatus according to (6), wherein
   the stimulation unit includes a multiplexer including a demultiplexer function for distributing the electric stimulation pulse signal to the plurality of stimulating electrodes, and a power supply circuit configured to supply power which stabilizes and operates the multiplexer, and
   the power supply circuit is driven by the alternating-current power supplied from the power supply unit.
(8) The living tissue stimulation apparatus according to (7), wherein
   the stimulating unit includes a rectifier circuit configured to rectify the alternating-current power to direct-current power, and a condenser that is connected to the power supply circuit and charged by the direct-current power which is generated by the rectifier circuit, and
   the condenser is charged to have a predetermined charged voltage when the alternating-current power from the power supply unit is supplied, and holds the state of the stimulation unit by the charged voltage when the alternating-current power is not supplied.
(9) The living tissue stimulation apparatus according to (8), wherein
   the stimulation unit includes a switch configured to switch a connection between the power supply circuit and the condenser, and a first control portion configured to perform an operation control of the switch based on whether or not the alternating-current power is supplied, and
   the first control portion turns on the switch and makes the condenser be charged when the alternating-current power is supplied from the power supply unit to the stimulation unit, and turns off the switch and holds the state of the stimulation unit by the charged voltage of the condenser when the alternating-current power is not supplied.
(10) The living tissue stimulation apparatus according to (9) further comprising a second control portion configured to generate a control signal for performing the operation control of the stimulation unit,
   wherein the periodic modulation circuit transmits the generated periodic-modulated wave to the stimulation unit via the cable, and intermittently supplies the periodic-modulated wave to the stimulation unit according to the output of the control signal from the second control portion.
(11) The living tissue stimulation apparatus according to (1) further comprising an imaging device adapted to image an object image, wherein the control signal is generated based on the object image.

### BRIEF DESCRIPTION OF THE DRAWINGS

Fig. 1 is a circuit block diagram of a living tissue stimulation apparatus.
Fig. 2 is an explanatory diagram of a configuration of a signal generation circuit.
Fig. 3 is an explanatory diagram of a relationship between a drive state of an alternating-current power supply and a state change of a multiplexer.
Fig. 4 is a timing chart of a control signal generation by the signal generation circuit.
Fig. 5 is a block diagram of a control system of a visual regeneration assisting apparatus.

### DESCRIPTION OF EXEMPLARY EMBODIMENTS

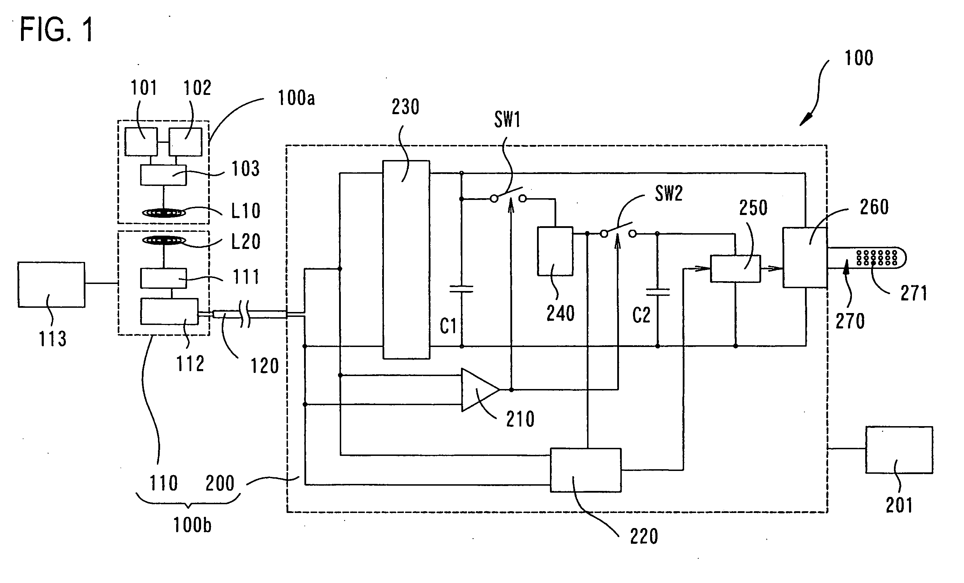

Exemplary embodiments will be described with reference to the drawings. Fig. 1 is a circuit block diagram of a living tissue stimulation apparatus 100. The living tissue stimulation apparatus 100 includes an external device 100a which is disposed outside of a body of a patient and an internal device 100b which is implanted inside of the body of the patient. The external device 100a generates information (a control signal) which is necessary in electric stimulation of the living tissue and supplies power for driving the entire living tissue stimulation apparatus 100. On the other hand, the internal device 100b performs the electric stimulation of the living tissue of a patient based on the supplied power and the information from the external device 100a.

Moreover, in the present embodiment, an external device control portion which performs the drive control of the entire external device 100a and an internal device control portion which performs the drive control of the entire internal device 100b are prepared. The internal device control portion includes a stimulation control portion (first control portion) 201 which performs the drive control of the entire stimulation unit 200, and a receiving control portion (second control portion) 113 which performs the drive control of the entire receiving unit 110.

The external device 100a includes a signal generation portion 101, an alternating-current power supply 102, a modulation circuit 103, and a transmission coil L10. In addition, the signal generation portion 101 generates a control signal for operating the external device 100b. As the control signal, there are data for an electrode designation signal, data for an electric stimulation pulse signal, and the like. Alternating-current power is supplied from the alternating-current power supply 102. The modulation circuit 103 modulates the alternating-current power based on the control signal and generates electromagnetic waves. The transmission coil L10 sends the electromagnetic waves which are output from the modulation circuit 103 to the internal device 100b side.

In addition, as the modulation method which modulates the alternating-current power to the electromagnetic waves by the modulation circuit 103, well-known modulation methods such as an amplitude modulation or a frequency modulation are used. Thereby, the sending and receiving of the electromagnetic waves suitable for a coil link with a receiver coil L20 described hereinafter is performed.

The internal device 100b includes a receiving unit (power supply unit) 110, stimulation unit 200, and a cable 120. The receiving unit 110 receives the electromagnetic waves which are sent from the external device 100a and extracts the power and the control signal. The stimulation unit 200 outputs an electric stimulation pulse signal for performing the electric stimulation of the living tissue. The cable 120 includes a plurality of conducting wires therein and electrically connects the receiving unit 110 and the stimulation unit 200.

The receiving unit 110 includes the receiver coil L20, a demodulator circuit 111, and a periodic modulation circuit 112. The receiver coil L20 receives the electromagnetic waves which are sent from the transmission coil L10. The demodulator circuit 111 demodulates the electromagnetic waves which are received by the receiver coil L20. The periodic modulation circuit 112 generates a periodic-modulated waver from a control signal which is obtained by the demodulation of the demodulator circuit 111 and power.

That is, the alternating-current power and the control signal are extracted by the demodulation of the demodulator circuit 111 from the received electromagnetic waves by the receiver coil L20, and the obtained alternating-current power is rectified to direct-current power by the rectifier circuit (illustration omitted). The direct-current power is used for driving the receiving unit 110 and as power which operates a decoder generating an electric stimulation pulse signal (stimulated current) output from electrodes 271 described hereinafter.

Moreover, in the present embodiment, when the state change of the stimulation unit 200 is generated, a periodic-modulated wave is generated by the periodic modulation of the periodic modulation circuit 112. In addition, the periodic-modulated wave which is generated by the periodic modulation is supplied to the stimulation unit 200 side via the cable 120. Moreover, the state change of the stimulation unit 200 is generated when switching the electrodes which output the electric stimulation pulse signal or when changing an output condition of the electric stimulation pulse signal.

Incidentally, periodic modulation refers to a modulation method which converts the control signal represented by a binary digit (0,1) to a combination of a waveform for each period of different periods (for example, 1.0 µs and 0.9 µs). Thereby, compared to an amplitude modulation which transmits the control signal as the amplitude change of the waveform, and a frequency modulation which converts the control signal to the frequency change of a carrier wave and transmits the frequency change, the transmission time of the control signal can be shortened and electric energy transmitted to the cable can be decreased in the periodic modulation.

Conducting wires which are formed of noble metals having good biocompatibility, for example, platinum, platinum iridium, stainless steel, titanium, or the like are bundled into one by a tube formed of an insulating resin having good biocompatibility, for example, a silicone, parylene, or the like, and the cable 120 is formed. A plurality of conducting wires are prepared in the cable 120. For example, a conducting wire (second conducting wire) for transmitting the power of the electric stimulation pulse signal and a conducting wire pair (first conducting wire pair) for transmitting the periodic-modulated waves which are generated by the periodic modulation, or the like are included in the cable. In addition, each conducting wire itself is also coated by an insulating resin having good biocompatibility such as parylene.

Moreover, in the present embodiment, a clock signal is generated by using the waveform period of the periodic-modulated wave (the detailed configuration will be described hereinafter). Thereby, the circuit configuration for generating the clock signal is not separately required, and the configuration becomes advantageous to miniaturization of the apparatus and low power consumption. In addition, also compared to other modulation methods such as the amplitude modulation in which the power transmission efficiency is decreased at the timing when the amplitude is decreased, and the frequency modulation and a phase modulation in which the configuration generating a clock signal which is a reference to the stimulation unit 200 side is indispensable, the clock signal is preferably generated by more simple configuration in the periodic modulation.

The receiving unit 110 having the above-described configuration is implanted to the temporal portion of a patient. On the other hand, the stimulation unit 200 is mounted on the sclera side or the like of the eyeball and electrically stimulates cells constituting the retina E1 from the sclera side (illustration omitted). The receiving unit 110 and the stimulation unit 200 are respectively separated from the living tissue by a well-known hermetic seal. Thereby, even though the alternating-current power (periodic-modulated wave) is supplied to each of the receiving unit 110 and the stimulation unit 200, influences such as noise or a leakage current to a living body are not easily generated.

On the other hand, after the cable 120 is extended toward a patient's eye under the skin along the temporal portion from the connection position with the receiving unit 110, the cable is placed on the eye socket through the inside of the upper eyelid of the patient. Moreover, the cable is connected to the stimulation unit 200 through the outside of the sclera (illustration omitted). In this way, since the cable 120 is wired along gaps or the like in the living body, it is required that the diameter is smaller and the cable is flexible. Thereby, like the receiving unit 110 or the stimulation unit 200, it is difficult to prevent the noise or the leakage current generated by the supply of the alternating-current power (periodic-modulated wave) through the hermetic seal. Therefore, since the present invention is configured such that power is intermediately provided to the cable 120 when necessary, influence of the noise which is generated when the alternating-current power passes through the cable is suppressed.

The stimulation unit 200 includes a power detection circuit 210, a signal generation circuit 220, a rectifier circuit 230, a stabilized power supply circuit (power supply circuit) 240, a control unit 250, a multiplexer 260, and a substrate 270. The power detection circuit 210 detects the amplitude of the periodic-modulated wave which is supplied from the receiving unit 110. The signal generation circuit 220 extracts a control signal from the periodic-modulated wave and generates a clock signal for obtaining the same period of (synchronized with) the stimulation unit 200. The rectifier circuit 230 rectifies the periodic-modulated wave (alternating-current power) to direct-current power. The stabilized power supply circuit (power supply circuit) 240 smoothes the power rectified by the rectifier circuit 230 and stabilizes the electric potential of the direct-current power. The control circuit 250 is driven by the power supply circuit 240 and generates a command signal (electrode designation signal or the like, electric stimulation pulse signal) based on the control signal (data for electrode designation signal, data for electric stimulation pulse signal, or the like) which is extracted by the signal generation circuit 220. The multiplexer 260 controls the electric stimulation pulse signal which is output from each electrode 271 based on the command signal from the control circuit 250. A plurality of electrodes 271, which are separately connected to the multiplexer 260 via the conducting wire (illustration omitted), are formed in the substrate 270.

The power detection circuit 210 is configured of a well-known comparator, is connected to the cable 120, and detects whether or not the amplitude of the periodic-modulated wave is present. The output side of the power detection circuit 210 is connected to switches SW1 and SW2. The stimulation control portion 201 switches each of switches SW1 and SW2 on or off depending on whether or not the amplitude of the periodic-modulated wave is detected. Specifically, the stimulation control portion 201 turns on the switches SW1 and SW2 when the periodic-modulated wave is detected. On the other hand, the stimulation control portion 201 turns off the switches SW1 and SW2 when the periodic-modulated wave is not detected.

The rectifier circuit 230 is configured of a bridge circuit or the like by a rectifier element such as a diode or a thyristor which are well-known, and is connected to the cable 120. The output of the rectifier circuit 230 is input to the power supply circuit 240 via the switch SW1. In addition, the output of the rectifier circuit 230 is also connected to the multiplexer 260. A condenser C1 is prepared between the output side of the rectifier circuit 230 and the input side of the power supply circuit 240; the switching of the connection between the condenser C 1 and the power supply circuit 240 (rectifier circuit 230) is performed by the switching operation of turning the switch SW1 on or off.

That is, since the switch SW1 is turned on when the periodic-modulated wave is supplied, the condenser C1 is charged by the direct-current power which is rectified by the rectifier circuit 230 and has a predetermined electric potential. On the other hand, when the periodic-modulated wave is not supplied, the switch SW1 is turned off, and therefore, the state of the stimulation unit 200 is held by the charging potential of the condenser C 1.

The power supply circuit 240 is configured of a constant voltage regulator or the like and stabilizes the amplitude of the direct-current power which is supplied from the rectifier circuit 230 to a constant value. The power supply circuit 240 is used as the drive power supply of the entire stimulation unit 200. Moreover, the output side of the power supply circuit 240 is connected to the control circuit 250, a condenser C2 is provided on the output side of the power supply circuit 240, and the switching of the connection between the condenser C2 and the power supply circuit 240 (control circuit 250) is performed by the switching operation of turning the switch SW2 on or off.

Thereby, since the switch SW2 is turned on when the periodic-modulated wave is supplied, the condenser C2 is charged by the output of the direct-current power from the power supply circuit 240 and has a predetermined electric potential. On the other hand, when the periodic-modulated wave is not supplied, since the switch SW2 is turned off, the state of the stimulation unit 200 is held by the charging potential of the condenser C2.

As described above, two condensers C1 and C2 are charged when the periodic-modulated wave is supplied to the stimulation unit 200 and the state of the stimulation unit 200 is held by the charging potentials of two condensers C 1 and C2 when the periodic-modulated wave is not supplied. Therefore, the state of the stimulation unit 200 (multiplexer 260) can be held regardless of whether or not power is supplied to the stimulation unit 200.

That is, since the state of the stimulation unit 200 is held by the power which is intermittently supplied according to the state change of the multiplexer 260, the power supply for holding the state of the stimulation unit 200 is not necessary. Thereby, influences of the noise and the leakage current which are generated by supplying power to the cable 120 are suppressed. In addition, the power of the external device 100a is efficiently used.

The control circuit 250 is driven by the direct-current power supplied from the power supply circuit 240 and generates a command signal (electrode designation signal or the like) based on the control signal (electrode designation signal data or the like) which is extracted by the signal generation circuit 220. The generated command signal becomes a signal which controls the operation of the multiplexer 260.

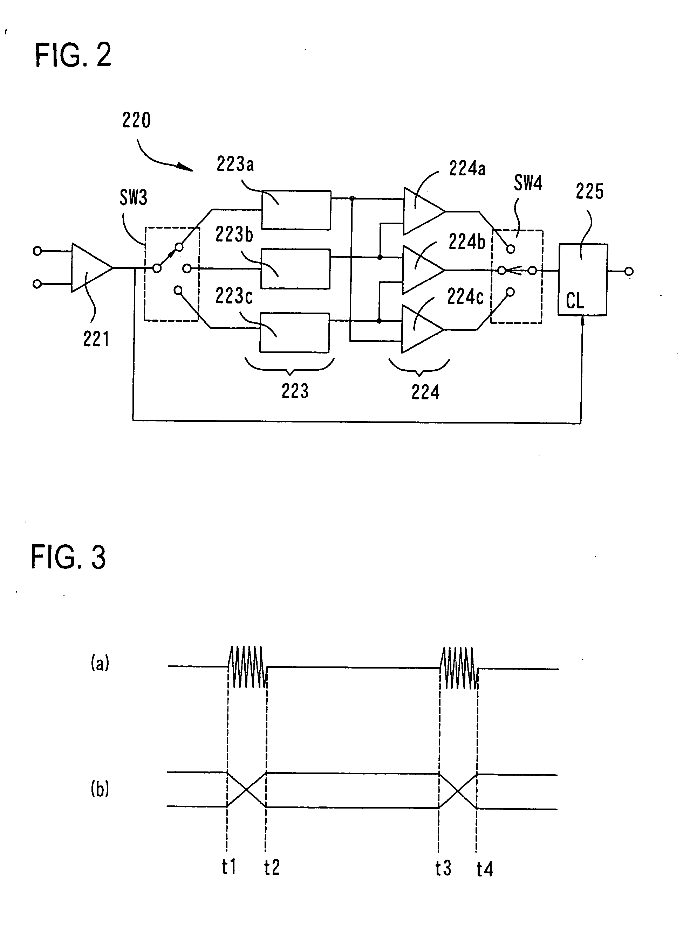

Next, the configuration of the signal generation circuit 220 will be described. Fig. 2 is an explanatory diagram of the configuration of the signal generation circuit. The signal generation circuit 220 includes a comparator 221, three period-voltage conversion circuits 223 (223a to 223c), three window comparators (hereinafter, referred to as a "comparator") 224, and a signal generation portion 225.

The comparator 221 extracts the waveform for each period by shaping the waveform of the periodic-modulated wave and generates a clock signal by detecting the period (for example, rising or falling) of the periodic-modulated wave. The period-voltage conversion circuit 223 converts the period of the periodic-modulated wave extracted by the comparator 221 to the voltage. The comparator 224 compares the output voltages which are continuously output from two conversion circuits among the three period-voltage conversion circuits 223, and outputs a predetermined signal. The signal generation portion 225 outputs a control signal of binary digits based on the output signal from the comparator 224 and the clock signal generated by the comparator 221.

Moreover, the comparator 221 and each of the period-voltage conversion circuits 223a to 223c are sequentially switched in the state of being connected to a switch SW3. In addition, the output of the comparator 221 is also connected to the signal generation portion 225, and the signal generation portion 225 generates a control signal based on the clock signal. In addition, two of period-voltage conversion circuits 223a to 223c are connected to each of comparators 224a to 224c respectively. Specifically, the period-voltage conversion circuits 223a and 223b are connected to the comparator 224a, the period-voltage conversion circuits 223b and 223c are connected to the comparator 224b, and the period-voltage conversion circuits 223c and 223a are connected to the comparator 224c. In addition, the connection state between each of comparators 224a to 224c and the signal generation portion 225 is sequentially switched by a switch SW4. Moreover, the switching timing of the switches SW3 and SW4 is performed based on the clock signal which is generated by the comparator 221.

According to the above-described configuration, if the waveform of one period is detected through the waveform shaping of the periodic-modulated wave by the comparator 221, the period information of one waveform which is extracted by the comparator 221 is converted to the voltage information by the period-voltage conversion circuit 223 which is connected by the switch SW3 (the circuit 223a is connected in Fig. 2). Specifically, the period-voltage conversion circuit 223 includes a condenser, and the condenser is charged to a predetermined voltage according to the waveform period (the voltage is determined for each period). Moreover, the obtained voltage information is input to the comparator 224. In addition, the control signal is obtained from the output signal of the comparator 224 in the signal generation portion 225.

In addition, the stimulation control portion 201 performs the switching operation of the switches SW3 and SW4 based on the clock signal. Moreover, the clock signal generated by the comparator 221 becomes a synchronous signal, which is necessary in that the stimulation control portion 201 and the control circuit 250 perform the drive control of the stimulation unit 200.

As described above, since the control signal and the clock signal are generated by using the period for each waveform of the periodic-modulated wave which is extracted by the comparator 221 of the signal generation circuit 220, it is not necessary to provide the circuit for generating the clock signal separately, which is advantageous in miniaturization of the internal device. In addition, power is efficiently used in the stimulation unit 200 in which limited power is supplied.

Moreover, in the above, three period-voltage conversion circuits 223 and three comparators 224 are provided respectively. However, three or more of period-voltage conversion circuits 223 and comparators 224 may be provided.

Here, operation of the signal generation circuit 220 will be explained in detail. By the control of the stimulation control portion 201, for example, in the first clock signal, the switch SW3 is connected to the period-voltage conversion circuit 223a and the switch SW4 is connected to the comparator 224b. Moreover, the waveform of one period of the periodic-modulated wave which is extracted by the comparator 221 is input to the period-voltage conversion circuit 223a, is converted to the voltage information for each period, and is input to the comparator 224a.

Next, the stimulation control portion 201 connects the switch SW3 and the period-voltage conversion circuit 223b by the next clock signal which is generated at the comparator 221, and connects the switch SW4 and the comparator 224c. Moreover, similarly, the voltage information of the next period which is converted by the period-voltage conversion circuit 223b is input to the comparator 224a.

In this way, if the voltage information of two periods is input to single comparator 224a, the comparator 224a detects whether or not there is a difference of the later voltage information (period information) with respect to the previous voltage information (period information) and generates a signal. For example, the comparator generates a signal of "0" when there is a voltage change (when there is a difference in the detected waveform periods), and the comparator generates a signal of "1" when there is no voltage change (when the detected waveform periods are the same as each other).

Moreover, if the switch SW4 is connected to the comparator 224a by the next clock signal which is generated at the comparator 221 through the stimulation control portion 201, the detection result of the signal by the comparator 224a is input to the signal generation portion 225. In the signal generation portion 225, in a state where the switching operation of the switch SW4 is sufficiently completed based on the clock signal, the signal which is formed from binary digits of "0" or "1" extracted by the comparator 224a is extracted as the control signal for the operation control of the multiplexer, and the signal is sent to the control circuit 250 at any time.

As described above, due to the fact that the operation switching of the switch SW3 and the switch SW4 is repeated based on the clock signal, the information (voltage information) of two waveforms for each period of the periodic-modulated wave generated by the periodic modulation is input to each of comparators 224a to 224c. Moreover, by detecting whether or not there is a change of the voltage information of two waveforms through the comparator 224, the control signal is extracted.

In addition, in the control circuit 250, a command signal is generated by collecting the control signal of binary digits, which are sent from the signal generation portion 225, with predetermined bit numbers. Moreover, the stimulation control portion 201 controls the switching of the state of the multiplexer 260 based on the command signal which is generated by the control circuit 250. In addition, the command signal includes a signal for switching the state of the entire multiplexer 260, a signal for designating the operating point, an electrode designation signal for designating the electrodes 271 which output the electric stimulation pulse signal, or the like. For example, the multiplexer 260 performs an operation or the like which makes the designated electrode 271 output the electric stimulation pulse signal based on the command signal.

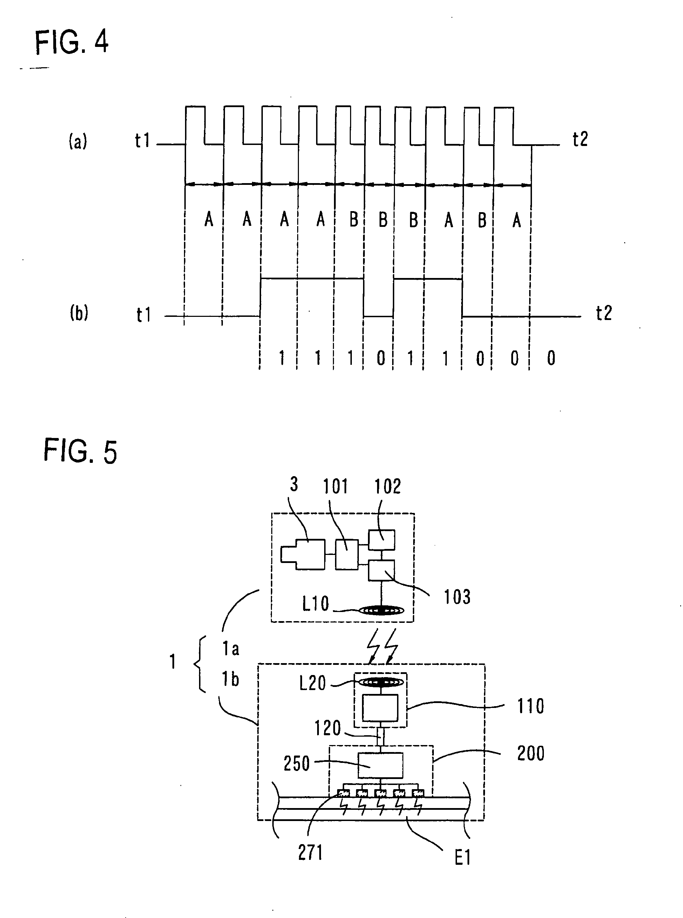

Next, the operation of the circuit including the above-described configuration will be described. Fig. 3 is an explanatory diagram of a relationship between the drive state of the periodic modulation circuit 112 and the state change of the multiplexer 260. Here, (a) of Fig. 3 shows the reception state of the periodic-modulated wave of the stimulation unit 200 and (b) of Fig. 3 shows the state change of the multiplexer 260. Fig. 4 is a timing chart for generating the control signal by the signal generation circuit 220, (a) of Fig. 4 shows the period of the waveform (for each waveform) of the periodic-modulated wave detected by the signal generation circuit 220, and (b) of Fig. 4 shows the control signal which is generated based on the detected results of the waveform period.

When the electromagnetic waves generated by the external device 100a are sent from the transmission coil L10 and received at the receiver coil L20(the receiving unit 110) of the internal device 100b, the electromagnetic waves are demodulated by the demodulator circuit 111 and the alternating-current power and the control signal are obtained. Moreover, when switching of the state of the stimulation unit 200 is performed, the periodic-modulated wave is generated based on the control signal by the periodic modulation circuit 112. That is, the periodic-modulated wave is supplied in the cable 120 as the power which drives the stimulation unit 200 only when the switching of the stimulation unit 200 is performed. Thereby, influence of noise on a living body is suppressed and the alternating-current power is efficiently used in the internal device 100b.

On the other hand, at the stimulation unit 200 side, it is detected whether or not the amplitude of the periodic-modulated wave in the power detection circuit 210 is present. Here, if the waveform of the periodic-modulated wave is extracted at time t1, the switches SW1 and SW2 are turned on, the periodic-modulated wave is rectified by the rectifier circuit 230, and the condenser C1 is charged. In addition, the condenser C2 is charged by the electric potential of the power supply circuit 240 and has a predetermined electric potential. Moreover, the multiplexer 260 is driven by the rectifier circuit 230; the entire stimulation unit 200 including the control circuit 250 and the multiplexer 260 is driven by the power supply circuit 240.

On the other hand, in the signal generation circuit 220, the waveform period for each period of the periodic-modulated wave is extracted by the comparator 221 and a clock signal is generated from the time t1. Moreover, the control signal is generated by comparison of the waveform period which is continuously extracted by the comparator 221. In addition, in (a) of Fig. 4, a period A is given when the waveform period which is detected by the signal generation circuit 220 is 1.0 µs and a period B is given when the waveform period is 0.9 µs. Moreover, as shown in (b) of Fig. 4, in the present embodiment, a signal of "1" is output when the waveform periods of the front and rear are the same as each other and a signal of "0" is output when the waveform periods in the front and rear are different from each other.

The generation method of the control signal by the signal generation circuit 220 is one example, a signal of "1" may be output when the waveform periods in the front and rear are different from each other and a signal of "0" may be output when the waveform periods are the same as each other. In addition, in the present embodiment, since the control signal is generated based on the comparison result of the period of two waveforms, the clock signal is delayed by two clocks and output in (b) of Fig. 4. Moreover, the generation of the control signal and the clock signal is continued until the waveform of the periodic-modulated wave is not detected at the time t2.

If the control signal (command signal) which is extracted as described above is input to the control circuit 250, the control circuit 250 collects the information of the control signal of binary digits, which are sequentially received, into a single command signal. In addition, the stimulation control portion 201 switches the state of the multiplexer 260 based on the command signal which is generated by the control circuit 250. On the other hand, the multiplexer 260 performs the operation control which makes the designated electrode 271 output the electric stimulation pulse signal based on the command signal.

On the other hand, if the periodic-modulated wave is not detected at the time t2, the stimulation control portion 201 turns off the switches SW1 and SW2 by the driving of the power detection circuit 210. Thereby, the circuit of the stimulation unit 200 comes to be in an opening state, and the state of the stimulation unit 200 (the control circuit 250 and the multiplexer 260) is held by the electric potential of the condensers C1 and C2 (refer to Fig. 3). That is, the state of the stimulation unit 200 is held even though power is not supplied from the receiving unit 110.

Similarly, when the periodic-modulated wave (alternating-current power) is supplied between time t3 and time t4, the state of the multiplexer 260 is switched, if the amplitude of the waveform of the periodic-modulated wave is not detected since the time t4, and the state of the control circuit 250 and the multiplexer 260 is held by the electric potential of the condensers C1 and C2. Thereby, the power supplied to the internal device 100b is efficiently used.

Next, as the living tissue stimulation apparatus including the above described configuration, a visual regeneration assisting apparatus which regenerates part or all of a patient's vision will be described as an example. Fig. 5 is a block diagram of a control system of a visual regeneration assisting apparatus. In addition, the same configurations as the living tissue stimulation apparatus 100 are denoted by the same reference numerals and explained.

The visual regeneration assisting apparatus 1 includes an external device 1a for imaging the outside world and an internal device 1b which applies electric stimulation to cells (refer to Fig. 5) constituting the retina E1 and stimulates regeneration of vision. The external device 1a includes a spectacles-shaped visor (illustration omitted) which is worn by a patient, an imaging device 3 which includes a CCD camera mounted on the visor or the like, a signal generation portion 101 which converts the subject image (image data) imaged by the imaging device 3 into the control signal, an alternating-current power supply 102 for supplying power to the internal device 1b, a modulation circuit 103 for modulating the alternating-current power based on the control signal, transmission coil L10 for sending the image data and the power generated by the external device to the internal device 1b, or the like. In addition, a magnet (illustration omitted) for fixing the position between the transmission coil L10 and the receiver coil L20 of the internal device 1b side described hereinafter is mounted on the center of the transmission coil L10.

The internal device 1b includes a receiving unit 110 which generates power and a stimulation signal from the electromagnetic waves received by the receiver coil L20, a stimulation unit 200 which electrically stimulates cells constituting the retina E 1 by an electric stimulation pulse signal output from a plurality of electrodes 271, and a cable 120, and the receiving unit 110 and the stimulation unit 200 are connected to each other via the cable 120. Moreover, since the diameter of the cable 120 of the present embodiment is formed so as to be smaller, the cable is appropriately disposed along the eyeball of a patient.

The receiving unit 110 generates a periodic-modulated wave by performing a periodic modulation to a portion of the power which is obtained when the switching of the state of the stimulation unit 200 (multiplexer 260) is performed, and sends the generated periodic-modulated wave to the stimulation unit 200 via the cable 120. That is, the alternating-current power is supplied to the stimulation unit 200 (multiplexer 260) only when performing the switching of the state of the stimulation unit.

The operation of the visual regeneration assisting apparatus 1 including the above-described configuration will be described. The object image (image data) imaged by the imaging device 3 is converted to a control signal by the signal generation portion 101. Moreover, the modulation circuit 103 performs an amplitude modulation of the alternating-current power from the alternating-current power supply 102 based on the control signal, and generates electromagnetic waves. The transmission coil L10 sends the generated electromagnetic waves to the internal device 1b side.

At the internal device 1b side, the electromagnetic waves received by the receiver coil L20 are demodulated by the receiving unit 110, and power and a control signal is obtained. At this time, the obtained power is used for the electric stimulation pulse signal output from the electrodes 271 and the operation of the receiving unit 110 itself. In addition, a portion of the power is periodically modulated based on the control signal (electrode designation signal data) only when the switching of the state of the stimulation unit 200 is performed and sent as a periodic-modulated wave. That is, the periodic-modulated wave (alternating-current power) is intermittently supplied in the cable 120 (only when necessary).

On the other hand, a control signal and a clock signal are extracted from the periodic-modulated wave in the stimulation unit 200 side and a command signal is generated. Therefore, the state of the multiplexer 260 is switched and the electric stimulation pulse signal generated from the remaining power which is obtained by the receiving unit 110 is output from the electrodes 271. Moreover, at the stimulation unit 200 side, a condenser (illustration omitted) is charged by using the power of the periodic-modulated wave, and the state of the stimulation unit is held regardless of whether or not the power is supplied.

As described above, the periodic-modulated wave (alternating-current power) is efficiently supplied via the cable only when the switching of the state of the stimulation unit is performed, influence of noise and occurrence risk of a leakage current on a living body are suppressed, and reliability of the apparatus is improved.

In addition, since the clock signal is generated by using the waveform period of the periodic-modulated wave, the circuit for generating the clock signal is not separately required at the simulation unit side, which becomes an advantageous configuration in miniaturization of the stimulation unit. Moreover, power is efficiently supplied to the multiplexer or the like.

In addition, in the present embodiment, power, the control signal, and the clock signal can be simultaneously obtained by the transmitting of the periodic-modulated wave which is generated by the periodic modulation. That is, since the information is transmitted by a pair of the conducting wires, the number of the wirings in the cable can be decreased, and the cable can be thinner and more flexible. Thereby, the cable is more appropriately disposed in a living body of a patient.

In addition, in the present embodiment, power is supplied from the alternating-current power supply of the outside to the internal device side. However, the present invention is not limited to this. The configuration of the present invention is applied even in a case where the power supply is prepared in the internal device side. Therefore, influence on a living body is suppressed, power is efficiently used, and the living tissue stimulation apparatus can be used in a living body over a more extended period of time.

Moreover, in the above, as the living tissue stimulation apparatus, the visual regeneration assisting apparatus is explained as the example. Except for that, the configuration of the present invention can be applied to well-known living tissue stimulation apparatuses, which are implanted in the body of a patient and perform electric stimulation, such as an artificial middle ear for transmitting sound vibrations to an auditory ossicle of a patient or a cardiac pacemaker which is implanted in the chest of a patient and suppresses occurrence of an irregular pulse by applying electrical stimulation to the heart. Thereby, the configuration of the living tissue stimulation apparatus becomes simpler and the power supplied from the power supply can be efficiently used, or the like, which becomes an advantageous configuration when implanting in the body for a long time.

## Claims

1. A living tissue stimulation apparatus comprising:
a power supply unit configured to generate alternating-current power and a first control signal for electric stimulation of a living tissue of a patient;
a periodic modulation circuit configured to periodically modulate the alternating-current power based on the first control signal to generate a periodic-modulated wave; and
a clock signal generation portion configured to detect a waveform for each period of the periodic-modulated wave and to generate a clock signal for operating the stimulation unit using the period of the periodic-modulated wave and to generate a second control signal from the periodic-modulated wave based on the clock signal,
a stimulation unit configured to be controlled based on the clock signal as a synchronous signal and to output an electric stimulation pulse signal through a plurality of electrodes that are configured to be disposed in a living body of the patient based on the alternating-current power and the second control signal to stimulate living tissue.

2. The living tissue stimulation apparatus according to claim 1 further comprising a cable configured to electrically connect the periodic modulation circuit to the clock signal generation portion,
wherein the cable includes a first conducting wire pair configured to transmit the periodic-modulated wave output from the periodic modulation circuit, and a second conducting wire configured to transmit the electric stimulation pulse signal, output from the electrodes, for electrically stimulating the living body.

3. The living tissue stimulation apparatus according to claim 2, wherein
the clock signal generation portion includes:
a comparator configured to detect the waveform period for each period of the periodic-modulated wave generated by the periodic modulation circuit and generate a clock signal based on the waveform period;
a plurality of period-voltage conversion circuits configured to convert the waveform period for each period of the periodic-modulated wave detected by the comparator into a voltage;
a plurality of window comparators configured to compare the voltages which are continuously output from the two period-voltage conversion circuits and output a signal; and
a signal generation portion configured to extract the clock signal based on the output signal from the window comparators.

4. The living tissue stimulation apparatus according to claim 3, wherein at least three period-voltage conversion circuits are provided and at least three window comparators are provided.

5. The living tissue stimulation apparatus according to claim 4, wherein the periodic-modulated wave output from the periodic modulation circuit is intermittently supplied to the stimulation unit via the first conducting wire pair when the state change of the stimulation unit is performed.

6. The living tissue stimulation apparatus according to claim 5, further comprising a power transmission unit configured to electrically connect the power supply unit to the stimulation unit,
wherein the stimulation unit is driven by the alternating-current power intermittently supplied from the power supply unit via the power transmission unit.

7. The living tissue stimulation apparatus according to claim 6, wherein
the stimulation unit includes a multiplexer including a demultiplexer function for distributing the electric stimulation pulse signal to the plurality of stimulating electrodes, and a power supply circuit configured to supply power which stabilizes and operates the multiplexer, and
the power supply circuit is driven by the alternating-current power supplied from the power supply unit.

8. The living tissue stimulation apparatus according to claim 7, wherein
the stimulating unit includes a rectifier circuit configured to rectify the alternating-current power to direct-current power, and a condenser that is connected to the power supply circuit and charged by the direct-current power which is generated by the rectifier circuit, and
the condenser is charged to have a predetermined charged voltage when the alternating-current power from the power supply unit is supplied, and holds the state of the stimulation unit by the charged voltage when the alternating-current power is not supplied.

9. The living tissue stimulation apparatus according to claim 8, wherein
the stimulation unit includes a switch configured to switch a connection between the power supply circuit and the condenser, and a first control portion configured to perform an operation control of the switch based on whether or not the alternating-current power is supplied, and
the first control portion turns on the switch and makes the condenser be charged when the alternating-current power is supplied from the power supply unit to the stimulation unit, and turns off the switch and holds the state of the stimulation unit by the charged voltage of the condenser when the alternating-current power is not supplied.

10. The living tissue stimulation apparatus according to claim 9 further comprising a second control portion configured to generate a control signal for performing the operation control of the stimulation unit,
wherein the periodic modulation circuit transmits the generated periodic-modulated wave to the stimulation unit via the cable, and intermittently supplies the periodic-modulated wave to the stimulation unit according to the output of the control signal from the second control portion.

11. The living tissue stimulation apparatus according to claim 1 further comprising an imaging device adapted to image an object image, wherein the control signal is generated based on the object image.

## Patentansprüche

1. Stimulationsvorrichtung für lebendes Gewebe, umfassend:
eine Energieversorgungseinheit, die zum Erzeigen von Wechselstromenergie und eines ersten Steuersignals für eine elektrische Stimulation eines lebenden Gewebes eines Patienten konfiguriert ist;
eine periodische Modulationsschaltung, die zum periodischen Modulieren der Wechselstromenergie basierend auf dem ersten Steuersignal konfiguriert ist, um eine periodisch modulierte Welle zu erzeugen;
einen Taktsignal-Erzeugungsbereich, der zum Erfassen einer Wellenform für jede Periode der periodisch modulierte Welle und zum Erzeugen eines Taktsignals zum Betätigen der Stimulationseinheit unter Verwendung der Periode der periodisch modulierten Welle und zum Erzeugen eines zweiten Steuersignals aus der periodisch modulierten Welle basierend auf dem Taktsignal konfiguriert ist,
eine Stimulationseinheit, die zum Steuern basierend auf dem Taktsignal als Synchronsignal und zum Ausgeben eines elektrischen Stimulationsimpulses durch eine Vielzahl von Elektroden konfiguriert ist, die zum Anordnen in einem lebenden Körper des Patienten konfiguriert sind, basierend auf der Wechselstromenergie und dem zweiten Steuersignal, um lebendes Gewebe zu stimulieren.

2. Stimulationsvorrichtung für lebendes Gewebe nach Anspruch 1, die ferner ein Kabel umfasst, das zum elektrischen Verbinden der periodischen Modulationsschaltung mit dem Taktsignal-Erzeugungsbereich konfiguriert ist,
wobei das Kabel ein Paar von ersten Leitungsdrähten, die zum Übertragen der von der periodischen Modulationsschaltung ausgegebenen periodisch modulierten Welle konfiguriert sind, und einen zweiten Leitungsdraht umfasst, der zum Übertragen des von den Elektroden ausgegebenen elektrischen Stimulationsimpulssignals konfiguriert ist, um den lebenden Körper elektrisch zu stimulieren.

3. Stimulationsvorrichtung für lebendes Gewebe nach Anspruch 2, wobei
der Taktsignal-Erzeugungsbereich umfasst:
einen Komparator, der zum Erfassen einer Wellenformperiode für jede Periode der von der periodischen Modulationsschaltung erzeugten periodisch modulierten Welle und zum Erzeugen eines Taktsignals basierend auf der Wellenformperiode konfiguriert ist;
eine Vielzahl von Periode-Spannung-Wandlerschaltungen, die zum Umwandeln der Wellenformperiode für jede Periode der vom Komparator erfassten periodisch modulierten Welle in eine Spannung konfiguriert sind;
eine Vielzahl von Fensterkomparatororen, die zum Vergleichen der Spannungen, die von den beiden Periode-Spannung-Wandlerschaltungen kontinuierlich ausgegeben werden, und zum Ausgeben eines Signals konfiguriert sind; und
einen Signalerzeugungsbereich, der zum Extrahieren des Taktsignals basierend auf dem Ausgabesignal von den Fensterkomparatoren konfiguriert ist.

4. Stimulationsvorrichtung für lebendes Gewebe nach Anspruch 3, wobei zumindest drei Periode-Spannung-Wandlerschaltungen vorgesehen sind und zumindest drei Fensterkomparatoren vorgesehen sind.

5. Stimulationsvorrichtung für lebendes Gewebe nach Anspruch 4, wobei die von der periodischen Modulationsschaltung der Stimulationseinheit ausgegebene periodisch modellierten Welle über das Paar der ersten Leitungsdrähte intermittierenden zugeführt wird, wenn die Zustandsänderung der Stimulationseinheit durchgeführt wird.

6. Stimulationsvorrichtung für lebendes Gewebe nach Anspruch 5, die ferner eine Energieübertragungseinheit aufweist, die zum elektrischen Verbinden der Energieversorgungseinheit mit der Stimulationseinheit konfiguriert ist,
wobei die Stimulationseinheit durch die von der Energieversorgungseinheit über die Energieübertragungseinheit intermittierend zugeführte Wechselstromenergie angetrieben wird.

7. Stimulationsvorrichtung für lebendes Gewebe nach Anspruch 6, wobei
die Stimulationseinheit einen Multiplexer mit einer Demultiplexer-Funktion zum Verteilen des elektrischen Stimulationsimpulssignals an die Vielzahl von Stimulationselektroden und eine Energieversorgungsschaltung umfasst, die zum Zuführen von Energie konfiguriert ist, die den Multiplexer stabilisiert und betätigt, und
die Energieversorgungsschaltung durch die von der Energieversorgungseinheit zugeführten Wechselstromenergie angetrieben wird.

8. Stimulationsvorrichtung für lebendes Gewebe nach Anspruch 7, wobei
die Stimulationseinheit eine Gleichrichterschaltung, die zum Gleichrichten der Wechselstromenergie in Gleichstromenergie konfiguriert ist, und einen Kondensator umfasst, der mit der Energieversorgungsschaltung verbunden ist und von der Gleichstromenergie aufgeladen wird, die von der Gleichrichterschaltung erzeugt wird, und
der Kondensator mit einer vorgegebenen Ladespannung aufgeladen wird, wenn die Wechselstromenergie von der Energieversorgungseinheit zugeführt wird, und den Zustand der Stimulationseinheit durch die Ladespannung hält, wenn die Wechselstromenergie nicht zugeführt wird.

9. Stimulationsvorrichtung für lebendes Gewebe nach Anspruch 8, wobei
die Stimulationseinheit einen Schalter, der zum Umschalten einer Verbindung zwischen der Energieversorgungsschaltung und dem Kondensator konfiguriert ist, und einen ersten Steuerbereich umfasst, der zum Durchführen einer Betätigungssteuerung des Schalters basierend darauf konfiguriert ist, ob die Wechselstromenergie zugeführt wird, oder nicht, und
der erste Steuerbereich den Schalter einschaltet und ein Laden des Kondensators bewirkt, wenn die Wechselstromenergie von der Energieversorgungseinheit der Stimulationseinheit zugeführt wird, und den Schalter ausschaltet und den Zustand der Stimulationseinheit durch die Ladespannung des Kondensators hält, wenn die Wechselstromenergie nicht zugeführt wird.

10. Stimulationsvorrichtung für lebendes Gewebe nach Anspruch 9, die ferner einen zweiten Steuerbereich umfasst, der zum Erzeugen eines Steuersignals zum Durchführen der Betätigungssteuerung der Stimulationseinheit konfiguriert ist,
wobei die periodische Modulationsschaltung die erzeugte periodisch modellierte Welle über das Kabel an die Stimulationseinheit überträgt, und die periodisch modulierte Welle der Stimulationseinheit gemäß der Ausgabe des Steuersignals vom zweiten Steuerbereich intermittierend zuführt.

11. Stimulationsvorrichtung für lebendes Gewebe nach Anspruch 1, die ferner eine Bildaufnahmevorrichtung aufweist, die zum Aufnehmen eines Objektbilds eingerichtet ist, wobei das Steuersignal basierend auf dem Objektbild erzeugt wird.

## Revendications

1. Appareil de stimulation de tissu vivant comprenant :
une unité d'alimentation configurée pour générer une puissance alternative et un premier signal de commande pour la stimulation électrique d'un tissu vivant d'un patient ;
un circuit de modulation périodique configuré pour moduler périodiquement la puissance alternative sur la base du premier signal de commande pour générer une onde modulée périodique ; et
une partie de génération de signal d'horloge configurée pour détecter une forme d'onde pendant chaque période de l'onde modulée périodique et pour générer un signal d'horloge pour mettre en oeuvre l'unité de stimulation en utilisant la période de l'onde modulée périodique et pour générer un deuxième signal de commande à partir de l'onde modulée périodique sur la base du signal d'horloge,
une unité de stimulation configurée pour être commandée sur la base du signal d'horloge en tant que signal synchrone et pour délivrer un signal impulsionnel de stimulation électrique par l'intermédiaire d'une pluralité d'électrodes qui sont configurées pour être disposées dans un corps vivant du patient sur la base de la puissance alternative et du deuxième signal de commande pour stimuler le tissu vivant.

2. Appareil de stimulation de tissu vivant selon la revendication 1, comprenant en outre un câble configuré pour connecter électriquement le circuit de modulation périodique à la partie de génération de signal d'horloge,
dans lequel le câble comprend une première paire de fils conducteurs configurés pour transmettre l'onde modulée périodique délivrée par le circuit de modulation périodique, et un deuxième fil conducteur configuré pour transmettre le signal impulsionnel de stimulation électrique, délivré par les électrodes, pour stimuler électriquement le corps vivant.

3. Appareil de stimulation de tissu vivant selon la revendication 2, dans lequel
la partie de génération de signal d'horloge comprend :
un comparateur configuré pour détecter la période de forme d'onde pendant chaque période de l'onde modulée périodique générée par le circuit de modulation périodique et pour générer un signal d'horloge sur la base de la période de forme d'onde ;
une pluralité de circuits de conversion période-tension configurés pour convertir la période de forme d'onde pendant chaque période de l'onde modulée périodique détectée par le comparateur en une tension ;
une pluralité de comparateurs à fenêtre configurés pour comparer les tensions qui sont délivrées continûment par les deux circuits de conversion période-tension et pour délivrer un signal ; et
une partie de génération de signal configurée pour extraire le signal d'horloge sur la base du signal de sortie des comparateurs à fenêtre.

4. Appareil de stimulation de tissu vivant selon la revendication 3, dans lequel au moins trois circuits de conversion période-tension sont prévus et au moins trois comparateurs à fenêtre sont prévus.

5. Appareil de stimulation de tissu vivant selon la revendication 4, dans lequel l'onde modulée périodique délivrée par le circuit de modulation périodique est appliquée par intermittence à l'unité de stimulation par l'intermédiaire de la première paire de fils conducteurs lorsque le changement d'état de l'unité de stimulation est effectué.

6. Appareil de stimulation de tissu vivant selon la revendication 5, comprenant en outre une unité de transmission de puissance configurée pour connecter électriquement l'unité d'alimentation à l'unité de stimulation,
dans lequel l'unité de stimulation est commandée par la puissance alternative délivrée par intermittence par l'unité d'alimentation par l'intermédiaire de l'unité de transmission de puissance.

7. Appareil de stimulation de tissu vivant selon la revendication 6, dans lequel
l'unité de stimulation comprend un multiplexeur comprenant une fonction de démultiplexeur pour répartir le signal impulsionnel de stimulation électrique vers la pluralité d'électrodes de stimulation, et un circuit d'alimentation configuré pour fournir une puissance qui stabilise et met en oeuvre le multiplexeur, et
le circuit d'alimentation est commandé par la puissance alternative délivrée par l'unité d'alimentation.

8. Appareil de stimulation de tissu vivant selon la revendication 7, dans lequel
l'unité de stimulation comprend un circuit de redressement configuré pour redresser la puissance alternative en une puissance continue, et un condensateur qui est connecté au circuit d'alimentation et chargé avec la puissance continue qui est générée par le circuit de redressement, et
le condensateur est chargé de manière à avoir une tension de charge prédéterminée lorsque la puissance alternative provenant de l'unité d'alimentation est délivrée, et maintient l'état de l'unité de stimulation par la tension de charge lorsque la puissance alternative n'est pas délivrée.

9. Appareil de stimulation de tissu vivant selon la revendication 8, dans lequel
l'unité de stimulation comprend un commutateur configuré pour commuter une connexion entre le circuit d'alimentation et le condensateur, et une première partie de commande configurée pour effectuer une commande d'actionnement du commutateur selon que la puissance alternative est délivrée ou non, et
la première partie de commande ferme le commutateur et provoque la charge du condensateur lorsque la puissance alternative est délivrée par l'unité d'alimentation à l'unité de stimulation, et ouvre le commutateur et maintient l'état de l'unité de stimulation par la tension de charge du condensateur lorsque la puissance alternative n'est pas délivrée.

10. Appareil de stimulation de tissu vivant selon la revendication 9, comprenant en outre une deuxième partie de commande configurée pour générer un signal de commande pour exécuter la commande de mise en oeuvre de l'unité de stimulation,
dans lequel le circuit de modulation périodique transmet l'onde modulée périodique générée à l'unité de stimulation par l'intermédiaire du câble, et délivre par intermittence l'onde modulée périodique à l'unité de stimulation en fonction de la sortie du signal de commande de la deuxième partie de commande.

11. Appareil de stimulation de tissu vivant selon la revendication 1, comprenant en outre un dispositif de formation d'image conçu pour former l'image d'une image d'objet, dans lequel le signal de commande est généré sur la base de l'image d'objet.
